# EUROPEAN PATENT SPECIFICATION

(11) **EP 3 488 013 B1**
(45) Date of publication and mention of the grant of the patent: **06.09.2023**
(21) Application number: 17745667.0
(22) Date of filing: 14.07.2017
(51) Int. Cl.: C12Q 1/6883, A61K 38/48, C12N 15/86, A61K 48/00, G01N 33/68

(54) **METHODS FOR DIAGNOSIS ALZHEIMER'S DISEASE AND VIRAL VECTORS FOR USE IN THERAPY THEREOF**
VERFAHREN ZUR DIAGNOSE VON MORBUS ALZHEIMER UND VIRALE VEKTOREN ZUR ANWENDUNG IN DER THERAPIE
PROCÉDÉS DE DIAGNOSTIC DE LA MALADIE D'ALZHEIMER ET VECTEURS VIRALES POUR UTILISATION DANS LE TRAITEMENT

(30) Priority: 20.07.2016 EP 16180434
(43) Date of publication of application: 29.05.2019
(73) Proprietor: École Polytechnique Fédérale de Lausanne (EPFL), 1015 Lausanne (CH)
(72) Inventor: SANCHEZ MUT, Jose Vicente, 1015 Lausanne (CH); GRÄFF, Johannes, 3013 Bern (CH); HEYN, Holger, 08908 Hospitalet de Llobregat - Barcelona (ES); ESTELLER BADOSA, Manel, 08908 Hospitalet de Llobregat - Barcelona (ES)
(74) Representative: Croce, Valeria
(86) International application number: PCT/EP2017/067848
(87) International publication number: WO 2018/015291

(56) References cited:
- WO-A2-2014/009733
- FR-A1- 2 929 292
- ARIANNA TUCCI ET AL: "Genetic variability at the PARK16 locus", EUROPEAN JOURNAL OF HUMAN GENETICS., vol. 18, no. 12, 4 August 2010 (2010-08-04), pages 1356-1359, XP055336047, CH ISSN: 1018-4813, DOI: 10.1038/ejhg.2010.125
- Dena Michelle ET AL: "GENETIC VARIATION AND DNA METHYLATION IN THE CONTEXT OF NEUROLOGICAL DISEASE", University College London , 10 June 2016 (2016-06-10), XP055335877, Retrieved from the Internet: URL:http://discovery.ucl.ac.uk/1485836/13/ Hernandez_Thesis_combined_compressed.pdf [retrieved on 2017-01-17]
- CORNELIS BLAUWENDRAAT ET AL: "Comprehensive promoter level expression quantitative trait loci analysis of the human frontal lobe", GENOME MEDICINE, vol. 8, 10 June 2016 (2016-06-10), page 65, XP055335421, DOI: 10.1186/s13073-016-0320-1
- ZHU XI-CHEN ET AL: "Association of Parkinson's Disease GWAS-Linked Loci with Alzheimer's Disease in Han Chinese", MOLECULAR NEUROBIOLOGY, HUMANA PRESS, US, vol. 54, no. 1, 6 January 2016 (2016-01-06), pages 308-318, XP036129041, ISSN: 0893-7648, DOI: 10.1007/S12035-015-9649-5 [retrieved on 2016-01-06]
- Lentz Thomas B. ET AL: "Viral vectors for gene delivery to the central nervous system", Neurobiology of Disease, vol. 48, no. 2, 1 November 2012 (2012-11-01), pages 179-188, XP55787434, AMSTERDAM, NL ISSN: 0969-9961, DOI: 10.1016/j.nbd.2011.09.014

## Description

### Background

Alzheimer's disease (AD) is the most common cause of dementia in western society. Despite intensive research efforts, its precise etiology remains unknown.

AD is believed to be caused by a combination of genetic and non-genetic risk factors, the latter likely being mediated by epigenetic mechanisms (Chouliaras L et al., Epigenetic regulation in the pathophysiology of Alzheimer's disease. Progress in neurobiology 2010, 90:498-510; Sanchez-Mut JV and Graeff J, Epigenetic Alterations in Alzheimer's disease. Frontiers In Behavioral Neuroscience 2015, 9:347). Genetic risk factors are usually interrogated by genome-wide association studies (GWAS) which have identified an important number of loci associated with AD pathology. However, GWAS tend to result in strong statistical associations of large portions of the genome, for which a causal relationship is difficult to establish. In contrast, locus-specific or epigenome-wide association studies (EWAS) identify locus-specific alterations providing mechanistic insights for a particular risk gene, but lack the statistical significance of GWAS (Rakyan VK et al., Epigenome-wide association studies for common human diseases. Nat Rev Genet. 2011, 12:529-41). Combining both approaches, it has recently become possible to identify single nucleotide polymorphisms (SNPs) that correlate with alterations in DNA methylation levels, named methylation quantitative trait loci (mQTLs), the importance of which for complex diseases is just starting to be recognized. As an example, mQTLs have been reported for cancer, osteoarthritis, multiple sclerosis, post-traumatic stress disorder and obsessive-compulsive and bipolar disorders but, thus far, not for neurodegenerative diseases such as AD, where there is still a need of new biomarkers and thus new methods of prognosis to better identify poor prognosis patients in AD.

Peptidase M20 domain containing 1 (PM20D1) is a protein coding gene that has been recurrently identified as a mQTL (IPDGC and WTCCC2, A Two-Stage Meta-Analysis Identifies Several New Loci for Parkinson's Disease. Plos Genet. 2011) and expression quantitative trait loci (eQTL) (Grundberg E et al., Global Analysis of the Impact of Environmental Perturbation on cis-Regulation of Gene Expression. Plos Genet. 2011). mQTL SNPs PM20D1 associated are known from Heyn H et al. 2013 (Heyn H et al., DNA methylation contributes to natural human variation. Genome Res. 2013, 23(9):1363-72).

Today, while the ultimate pathology of Alzheimer's disease is fairly well established, effective diagnostic methods and treatment modalities remain in fact elusive because of the complex biological basis for the etiology and pathogenesis of the disease. Clinical diagnostic techniques for Alzheimer's disease currently rely on screening individuals displaying symptoms of dementia by excluding other possible causes such as depression, poor nutrition, other dementing conditions (e.g., Parkinson's disease with dementia), or drug interactions. These qualitative and unspecific methods often leave AD misdiagnosed or unrecognized until later stages in the disease when treatments may be less effective. Genetic testing have been developed so far, useful in defining clinical trial project but not 100% capable to test for pathology at a single individual level (Albertici A et al., Clinical, genetic, and neuroimaging features of Early Onset Alzheimer Disease: the challenges of diagnosis and treatment. Curr. Alzheimer Res. 2014, 11 (10) :909-17). Imaging techniques are also largely applied in AD testing (Schindler SE et al., Advances in diagnostic testing for Alzheimer disease. Mo Med. 2013, 110(5):401-5).

WO 2014/009733 discloses therapeutic targets for Alzheimer's disease. FR-A-2929292 discloses detection of Alzheimer's disease using genetic alterations or RNA expression.

There is therefore a need for specific diagnostic tests and biomarkers that are predictive of a subject developing AD and that can help establish a clear prognosis of the disease.

### Description

Various embodiments of the present description provide methods for the e the diagnosis, and/or prognosis of AD. Further areas of applicability will become apparent from the description provided herein. It should be understood that the description and specific examples are intended for purposes of illustration only and are not intended to limit the scope of the present teachings.

### Figure Description

The drawing figures described herein are for illustration purposes only and are not intended to limit the scope of the present teachings in any way. The present teachings will become more fully understood from the detailed description and the accompanying drawing figures wherein:

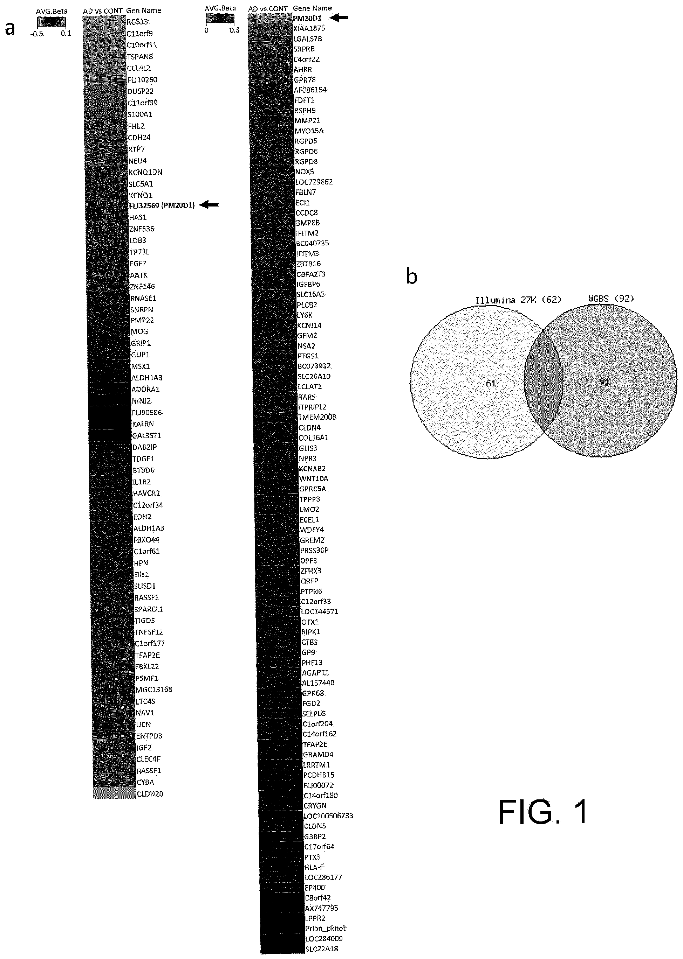

Figure 1: a) Heatmap of differentially methylated genes identified in the GSE45775 Illumina 27K DNA methylation arrays and hippocampus samples of AD (left), and GSE57361 Whole genome Bisulphite sequencing (WGBS) and frontal cortex samples of AD (right); b) Venn diagram showing that only PM20D1 is commonly hyper methylated in both studies.

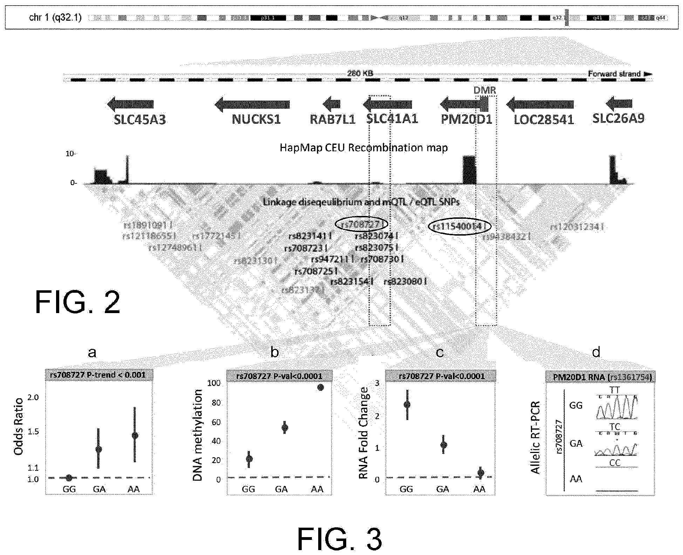

Figure 2: overview of the rs708727-PM20D1 locus. The region comprises several genes, indicated by arrows. Below the genes, the rate of recombination peaks in the gene body of PM20D1 dividing rs708727 and the PM20D1 promoter in two independent linkage-disequilibrium blocks, according to the HapMap CEU recombination map indicated below. rs708727 (circled), located in exon six of the neighbouring gene SLC41A1, is in linkage-disequilibrium with other SNPs of the block, among which rs708730, located on the same SCL41A1 gene. rs11540014 (circled), located in the promoter of PM20D1, is in linkage disequilibrium with other SNPs of its own block. The differentially methylated region (DMR) on PM20D1 promoter is highlighted in black.
Figure 3: a) rs708727 shows an allele-dose dependent association with AD; b) rs708727 correlates with the levels of PM20D1 DNA methylation in human brain samples measured by pyrosequencing; c) rs708727 correlates with PM20D1 expression in human brain samples measured by quantitative Real-Time PCR; d) rs1361754 is located in the coding region of PM20D1 and it is in linkage disequilibrium with rs708727. Retro transcription PCR Sanger sequencing of rs1361754 heterozygous samples detects rs1361754 (T) but not rs1361754 (A) RNA transcripts, indicating that only the rs708727 (G) chromatid is active.

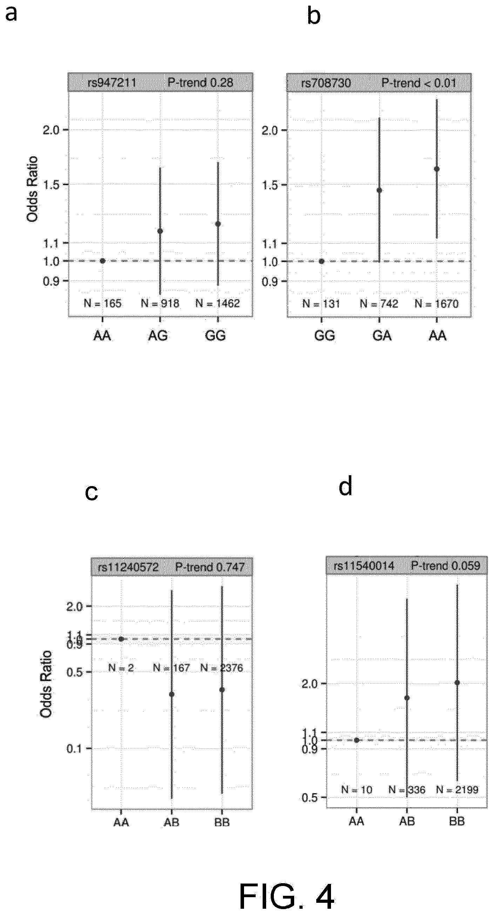

Figure 4: AD association analysis of SNPs located in both linkage-disequilibrium blocks of rs708727-PM20D1 promoter region, a), b) mQTL SNPs located in the rs708727 linkage-disequilibrium region are also associated with AD. c), d) SNPs located in PM20D1 promoter linkage-disequilibrium region are not associated with AD.

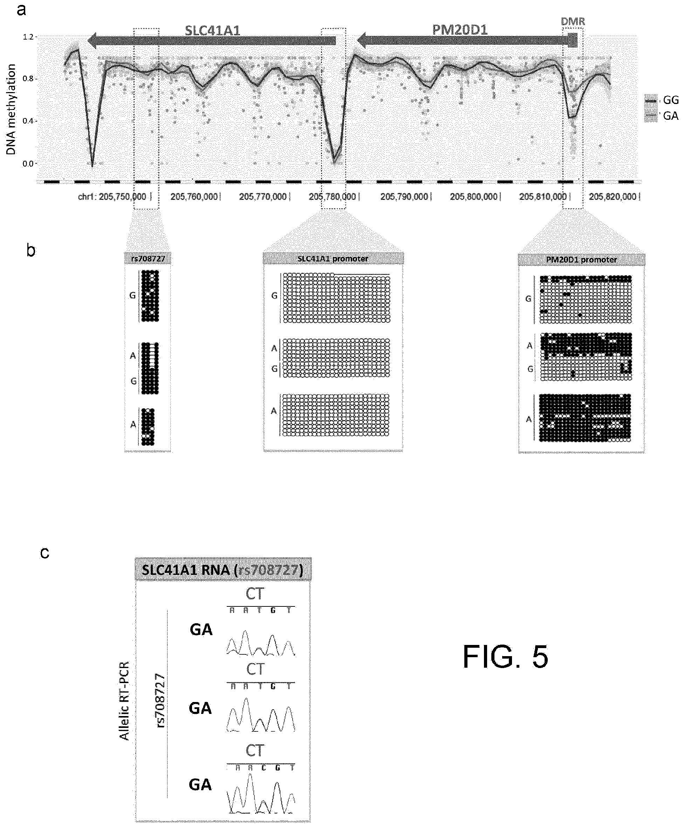

Figure 5: a) whole genome bisulfite sequencing analysis of DNA methylation in GG and GA rs708727 human frontal cortex samples. Differences in DNA methylation are restricted to the DMR detected in PM20D1 promoter; b) locus-specific bisulfite sequencing of the rs708727, SLC41A1 and PM20D1 promoter regions confirming the whole genome bisulfite sequencing and pyrosequencing data in GG, GA and AA rs707827 human frontal cortex samples; c) SLC41A1 expression from three different heterozygous samples, results obtained by RT-PCR Sanger sequencing.

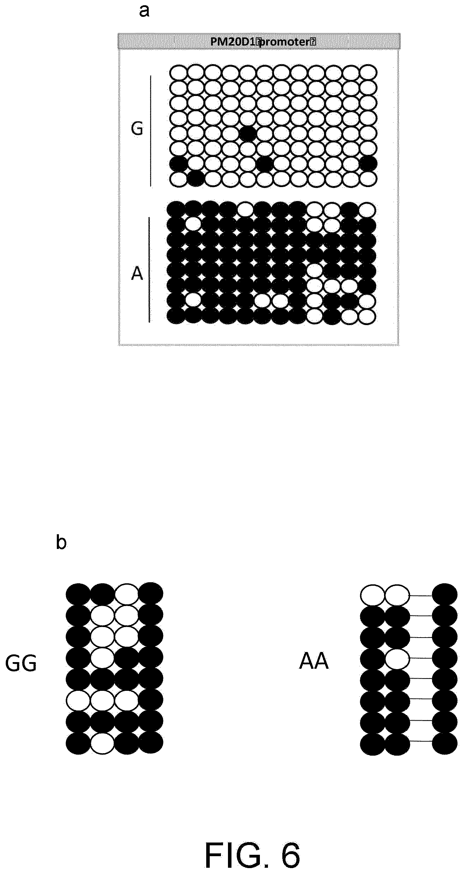

Figure 6: a) PM20D1 promoter DNA methylation is correlated with rs708727 genotypes in immortalized B cells measured by bisulfite sequencing/cloning; b) rs708727 region DNA methylation is not correlated with rs708727 genotypes in immortalized B cells measured by bisulfite sequencing/cloning.

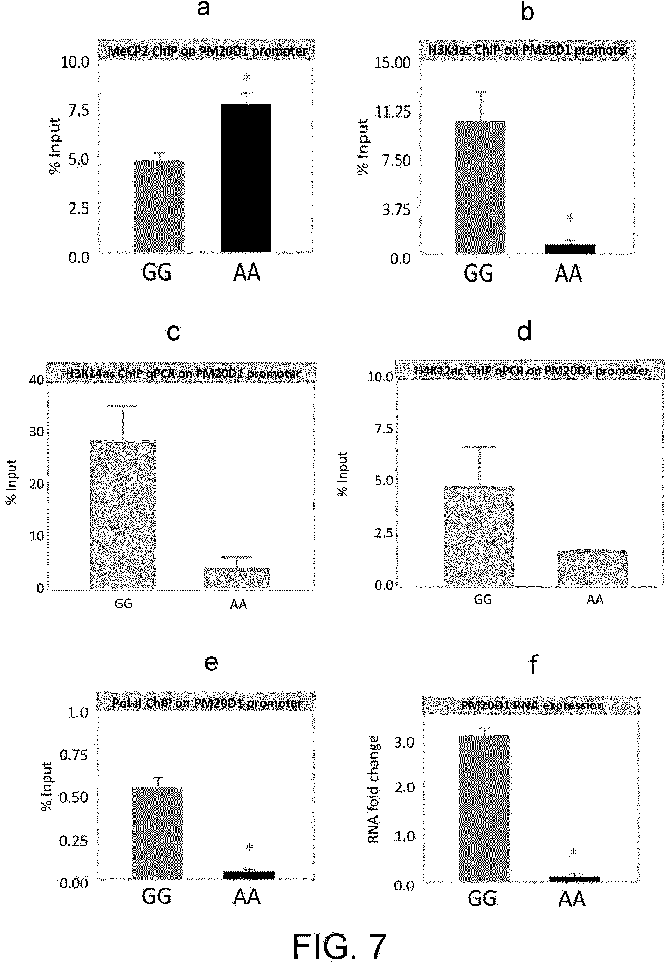

Figure 7: a) Chromatin immunoprecipitation assay (ChIP) showing higher binding of MeCP2 in rs708727 AA methylated samples on the PM20D1 promoter region; b) ChIP showing that PM20D1 promoter region of rs708727 AA samples is depleted of H3K9ac; c) ChIP showing that PM20D1 promoter region of rs708727 AA samples is depleted of H3K14ac; d) ChIP showing that PM20D1 promoter region of rs708727 AA samples is depleted of H4K12ac; e) ChIP showing lower binding of Polymerase-II in the PM20D1 promoter; f) qRT-PCR showing lower PM20D1 RNA levels in rs708727 AA samples.

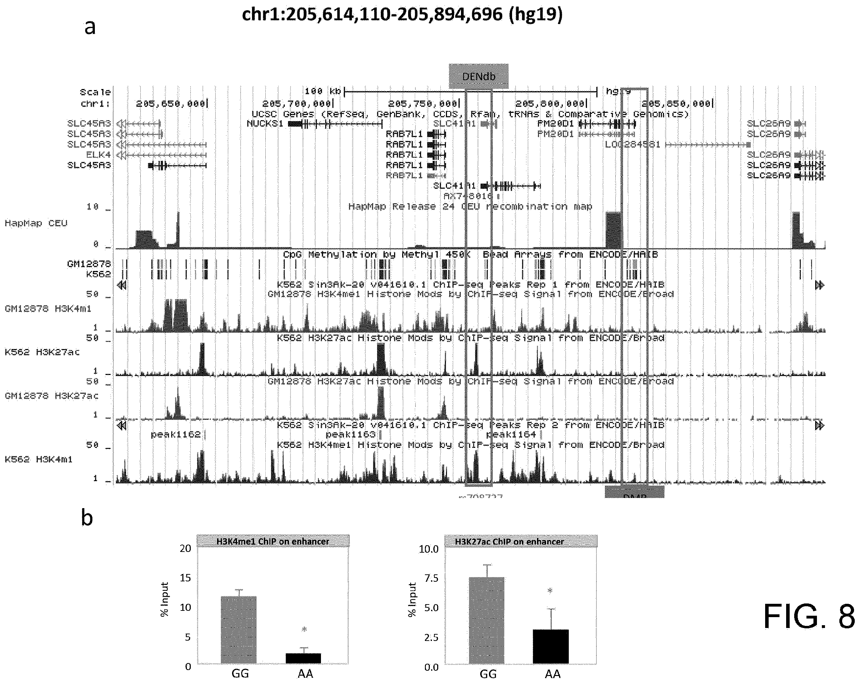

Figure 8: a) enhancer-enriched epigenetic marks such as histone 3 lysine 4 mono-methylation (H3K4me1) and H3K27ac in cells lines for which the DNA methylation profile was also available using ENCODE (Encyclopedia of DNA Elements; ENCODE/Broad Histone Track for H3K4me1 and H3K27ac, and HAIB Methyl450 Track for K562 and GS12878 cell lines) showing a peak of H3K4me1 and H3K27ac at 10kb downstream of rs708727 in the PM20D1 promoter non-methylated K562 cells, suggesting the existence of allele-dependent active enhancer. b) ChIP assay showing that H3K4me1 (left) and H3K27ac (right) are enriched in rs708727 (GG) samples in the rs708727-enhancer locus.

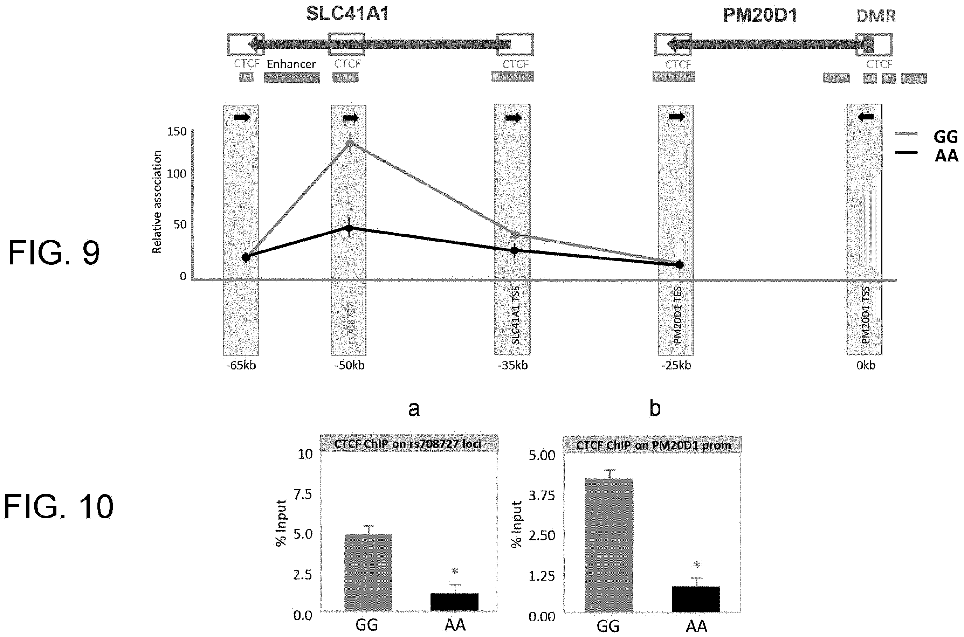

Figure 9: relative interaction frequencies between regions as a function of the genomic distances (in Kb) and rs708727 genotype.
Figure 10: ChIP assay showing the binding of CTCF on rs708727 loci (a) and on PM20D1 promoter region (b).

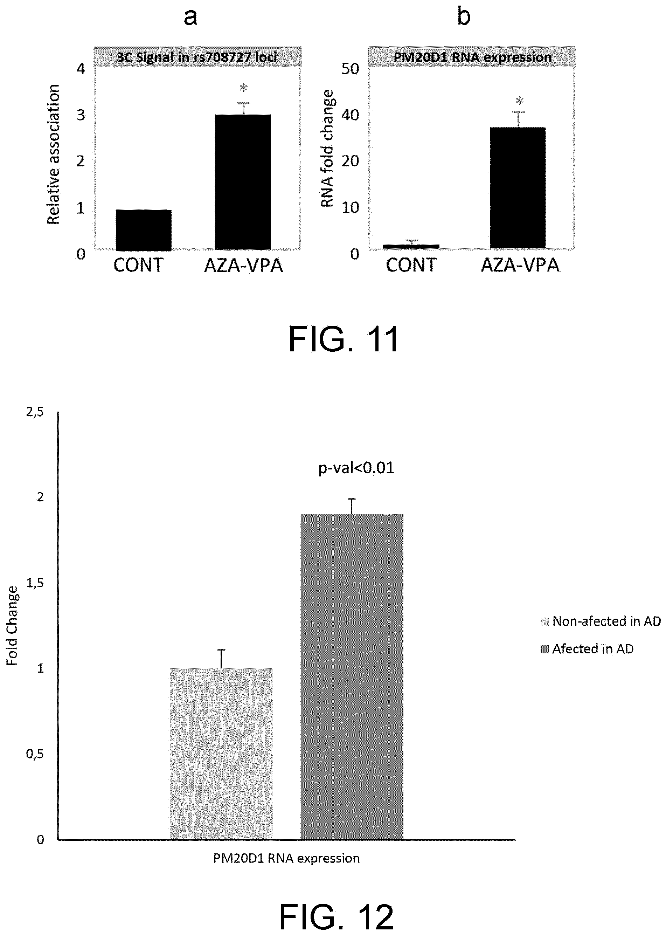

Figure 11: a combination of 5-aza/VPA restores 3C DNA (a) and PM20D1 expression levels (b).
Figure 12: PM20D1 RNA expression in AD-affected and non-affected mouse brain areas.

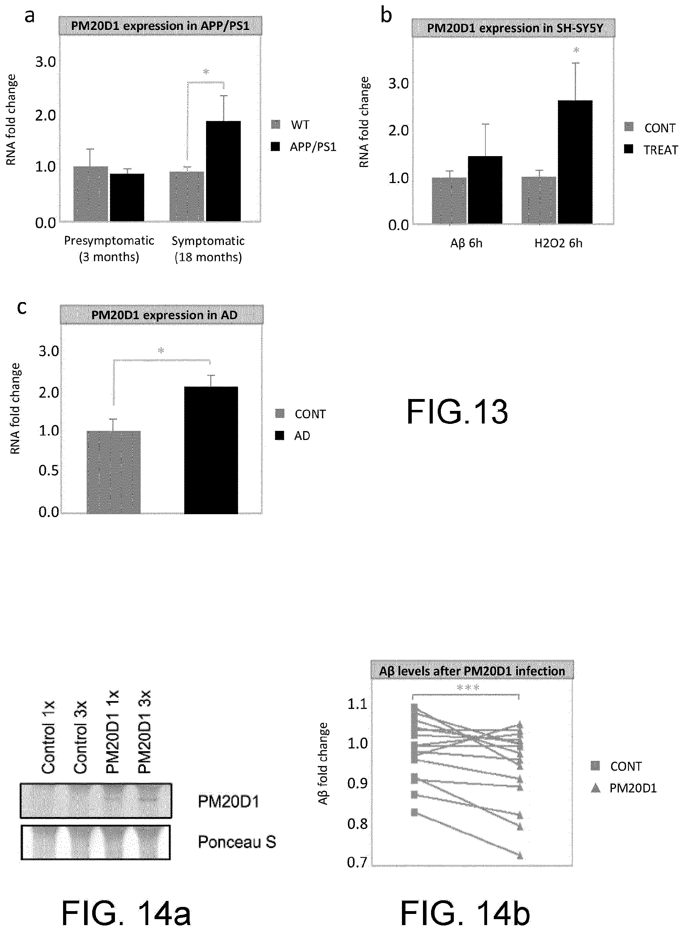

Figure 13: PM20D1 RNA expression a) in wt and APP/PS1 mice; b) in neuroblastoma cells, following exposure to AD-related stressors such as reactive oxygen species produced by H2O2 and Aβ; c) in rs708727 genotype-corrected frontal cortex of human AD samples. Data are presented as means plus standard error. *p<0.05 by student's t-test.
Figure 14: a) overexpressed PM20D1 in hippocampal primary culture is detected in culture media; b) PM20D1 transduction decreases Ab levels in hippocampal primary cultures. Data represent 15 different paired experiments performed (mock versus PM20D1 transduction) with three different virus batch productions (five experiments per virus batch). ***p<0.001; paired t-test.

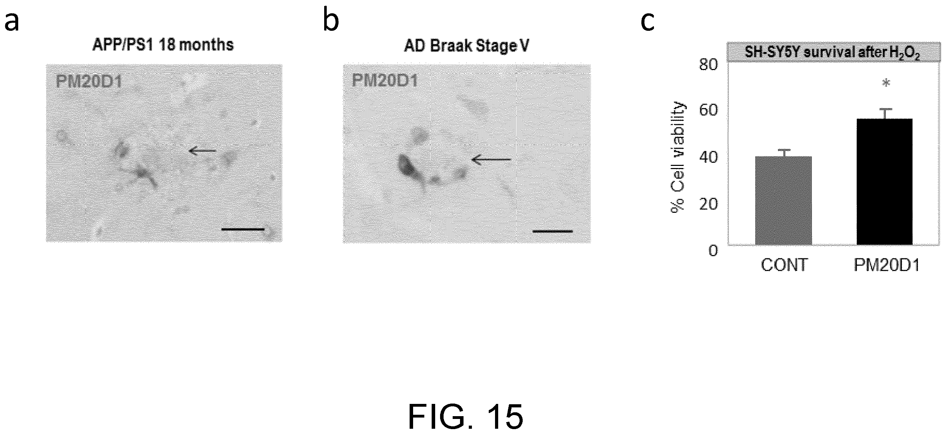

Figure 15: a) Symptomatic APP/PS1 mice (18 months of age) and b) postmortem Alzheimer's disease (AD) at advanced disease stages (Braak V) show increased PM20D1 immunoreactivity in glial cells surronding neuritic amyloid plaques; c) PM20D1 overexpression in SH-SY5Y cells increases cell survival upon H₂O₂ treatment.

### Correlation observed in human samples

The inventors analyzed the epigenetic profile (methylation profile of the histones) in an enlarged cohort of AD patients. Hippocampus and frontal cortex of AD samples have been analyzed. EWAS studies surprisingly identified only a single DNA hyper methylation common to hippocampus and frontal cortex samples analyzed, consisting in a hyper methylation of PM20D1, as reported in Figure 1.

Following from this observation, previously associated PM20D1 mQTL SNPs (Heyn et al. 2013, previously cited), reported in Figure 2, have been selected to evaluate their relation, if any, with AD. Data from a public available database from NCBI GWAS studies (phs000168.v1.p1 NIA) have been examined by fitting a Bayesian univariate logistic regression with phenotype as outcome. To note, the analyzed data were obtained in blood sample. A significant allele-dose dependent association of SNP rs708727 with the odds ratio for developing AD has been detected (P-trend <0.001, Figure 3a). Supporting this observation, four other reported PM20D1 mQTL and eQTL SNPs, located in the linkage-disequilibrium region of rs708727, rs947211, rs708730, rs708724 and rs823130, also correlated with AD (Figure 4a and b; Table 1, bold), albeit not as strongly.

**Table 1: AD association analysis of eQTLs from GTEX Consortium 2015, Science, included in the phs000168.v1.p1 NIA - Late Onset AD and National Cell Repository for AD Family Study. Major and minor alleles and linkage disequilibrium data from SNAP Broad Institute database are also indicated.**

| **SNP** | **Distance** | **RSquared** | **DPrime** | **Major** | **Minor** | **LD Block** | **eQTL Effect Size** | **eQTL P-Value** | **AD p.value** | **AD model** |
|---|---|---|---|---|---|---|---|---|---|---|
| **rs823130** | 53513 | 0,745 | 0,892 | T | C | SNP | -0,8 | 5,60E-35 | 0,02738 | additive |
| **rs708724** | 24222 | 0,838 | 0,931 | C | A | SNP | -0,78 | 1,80E-32 | 0,01599 | additive |
| **rs947211** | 15220 | 0,258 | 1 | G | A | SNP | -0,42 | 1,6E-06 | 0,28850 | additive |
| **rs708727** | 0 | 1 | 1 | A | G | SNP | -0,92 | 1,70E-49 | 0,00076 | additive |
| **rs708730** | 9895 | 0,137 | 1 | A | G | SNP | -0,45 | 6,2E-06 | 0,00990 | additive |
| rs1361754 | 33987 | 0,666 | 1 | G | A | SNP | -0,77 | 5,40E-25 | - | - |
| rs11240572 | 11091 | 0,002 | 1 | C | A | Recomb site | - | - | 0,75510 | additive |
| rs11540014 | 0 | 1 | 1 | T | C | promoter | - | - | 0,05884 | additive |
| rs12031234 | 59584 | 0, 908 | 1 | T | G | promoter | - | - | 0,07426 | additive |

Contrarily, SNPs located in the PM20D1 promoter linkage-disequilibrium region were not associated with AD (Figure 4c and d; underlined in Table 1). Remarkably, rs708727, rs947211, rs708730, rs708724 and rs823130, and the PM20D1 promoter region are located in two independent linkage disequilibrium regions (HapMap Figure 2), therefore excluding the mQTL/eQTL association to be the mere consequence of a linkage-disequilibrium effect.

By testing frontal cortex sample, a strong correlation in a rs708727 haplotype and dose-dependent manner with PM20D1 DNA methylation has been observed (Figure 3b, P-val<0.0001; Pearson correlation) but also with PM20D1 RNA levels (Figure 3c, P-val<0.0001; Pearson correlation). Similar associations have been observed in different tissues at RNA levels (data not shown) suggesting that the correlation is independent from the cell origin. Furthermore, PM20D1 is monoallelically expressed and it is only detectable from rs708727 G chromatids in heterozygous samples (Figure 3d). As a control, SLC41A1 expression has been evaluated, since rs708727 lies in the coding region of SLC41A1 (Figure 2). SLC41A1 does not show differences in DNA methylation and its RNA can be detected from both chromatids (Figure 5a, b, c). Taken together, these findings surprisingly indicate that PM20D1 methylation and expression are dependent on the rs708727 SNP, and that both, the epigenetic regulation of PM20D1 and the genetic variation at rs708727, are linked to AD.

### Assays on immortalized B cells

B cells have been immortalized from rs708727 GG and AA carriers. Similar to the frontal cortex samples, rs708727 genotype was associated with DNA methylation levels of PM20D1 in a haplotype dose-dependent manner (Figure 6a), while the DNA methylation in the rs708727 region did not differ between genotypes (Figure 6b). *Methyl CpG Binding Protein 2 (MeCP2)* binding has been then evaluated and higher binding levels were observed in rs708727 AA carriers (Figure 7a), wherein rs708727 AA carriers showed lower levels of histone 3 lysine 9 acetylation (H3K9ac) (Figure 7b) and of H3K14ac and H4K12ac (Figure 7c and d), which were accompanied by lower RNA polymerase-II accessibility (Figure 7e) and reduced PM20D1 RNA expression (Figure 7f), in agreement with the known interaction of MECP2 with histone deacetylase 1 (HDAC1; Suzuki et al. Direct association between PU.1 and MeCP2 that recruits mSin3A-HDAC complex for PU.1-mediated transcriptional repression. Oncogene 2003, 22).

The genetic region of chromosome 1 encompassing rs708727 and PM20D1 has been then screened for enhancer-enriched epigenetic marks such as histone 3 lysine 4 mono-methylation (H3K4me1) and H3K27ac in cells lines for which the DNA methylation profile was also available using ENCODE (Encyclopedia of DNA Elements; ENCODE/Broad Histone Track for H3K4mel and H3K27ac, and HAIB Methyl450 Track for K562 and GS12878 cell lines) . A peak of H3K4me1 and H3K27ac has been detected at 10kb downstream of rs708727 in the PM20D1 promoter non-methylated K562 cells, which posits the existence of allele-dependent active enhancer (Figure 8a). Supporting this observation, an enrichment of H3K4me1 and H3K27ac has been observed in rs708727 GG samples (Figure 8b). Considering that enhancers often physically interact with their target promoters (Whalen S et al. Enhancer-promoter interactions are encoded by complex genomic signatures on looping chromatin. Nature Genetics 2016, 48:488-496), a chromatin conformation capture assay (3C) has been performed in rs708727 GG (square) and AA (circle) carrier B cells. PM20D1 promoter region has been found to interact with rs708727 region, but also this interaction was rs708727 genotype-dependent and weakened in the PM20D1 transcriptionally silent genotype (AA) (Figure 9). In line, CCCTC-binding factor (CTFC), which is responsible for more than 90% of DNA loops in mammalian genomes and negatively affected by DNA methylation, showed reduced binding to the rs708727 and PM20D1 regions in AA carriers (Figure 10a, b).

B cells were then treated with the DNA demethylating agent 5-Azacytidine-dC (5-aza) in combination with the histone deacetylase inhibitor valproic acid (VPA). 5-aza/VPA treatment restored CTFC binding and allowed for the rs708727-PM20D1 chromatin loop to reform (Figure 11a). Moreover, the treatment increased PM20D1 RNA expression (Figure 11b).

The reported experiments clearly demonstrate a genotype-dependent three-dimensional chromatin interaction between the rs708727 region, with enhancer-like characteristics, and the promoter region of PM20D1, which display a genotype and loop-dependent differential expression.

### Correlation observed in AD mouse model

The extensively used APP/PS1 mouse model of AD has been used. According to Allen Brain Atlas mouse database, AD affected and non-affected brain area have been separated and tested for PM20D1 RNA expression levels. The obtained data are reported in Figure 12, indicating an higher expression in AD affected brain regions.

Frontal cortex has then been analyzed in wt and APP/PS1 mice, at 3 and 18 months. At 3 months, the APP/PS1 mice are presymptomatic and no differences in PM20D1 expression are observed. At 18 months, the APP/PS1 mice are symptomatic and a significative difference is observed, where PM20D1 expression is increased in AD (Figure 13a and Figure 15a), showing the strongest immunoreactivity for PM20D1 the glial cell surrounding the neuritic amyloid plaques (Figure 15a).

### Assay in SH-SY5Y neuroblastoma cells

SH-SY5Y cells were exposed to stimuli extensively used to mimic AD intracerebral insult, i.e. oxidative stress and Amyloid-β. Cells were treated with 0.02% H₂O₂ or with 50uM Aβ during 6 hours. Before and after treatment, PM20D1 expression levels were evaluated. The results, reported in Figure 13b, indicate a stimuli-induced increase in PM20D1 expression.

### PM20D1 overexpression increases cell survival

PM20D1 was overexpressed in SH-SY5Y cells then exposed to 0.02% H₂O₂ stimuli. Cell viability was evaluated. Data, shown in Figure 15c, demonstrates that the overexpression of PM20D1 increases cell survival upon H₂O₂ treatment.

These data suggest that PM20D1 is increased in glial cells in response to the presence of neuritic amyloid plaques and might increase neuronal survival in response to reactive oxygen species that are associated with AD.

### PM20D1 expression in human samples

PM20D1 RNA expression was analyzed in a publicly available database from NCBI expression studies (GSE330000). The represented AD-samples showed lower levels of PM20D1 with respect to control samples (log.Fold change -0.0195; P-val<0.05). This data confirm the correlation AA rs708727 and AD, wherein PM20D1 is monoallelically expressed and it is only detectable from rs708727 G chromatids in heterozygous samples (Figure 3d). The collected and genotyped frontal cortex samples were then analysed. After genotype correction, an increase of PM20D1 expression was observed in AD samples, in line with the observation in in vitro and AD mouse model (Figure 13c) and demonstrating that G rs708727 carriers upregulate PM20D1 in AD.

When PM20D1 has been evaluated in postmortem Alzheimer's disease (AD) at advanced disease stages (Braak V), an increased PM20D1 immunoreactivity in glial cells surronding neuritic amyloid plaques has been observed (Figure 15b).

### Aβ levels following PM20D1 overexpression

Primary hippocampal cultures obtained from P0 APPswe/PSldE9 mice (Borchelt et al. 1997) were cultured in media consisting of Neurobasal (Invitrogen), B27 supplement (Invitrogen), L-glutamine (Invitrogen) and penicillin/streptomycin (Invitrogen) (0.2 ml per well) on 96 wells plates (2.5 × 10^4 cells/well) coated with Cultrex poly-L-lysine (Trevigen). At days-in-vitro (DIV) 5, cells were treated with cytosine β-D-arabinofuranoside (AraC, 2nM) to inhibit glial cell division and infected at DIV5 with 10-40 × 10^3 (100-400 ng/well) viral particles containing either a mock or PM20D1 version of the plasmid. At DIV12 media from 96 wells was collected and measured using the Aβ 40 ELISA kit, human (Novex). PM20D1 contains a N-terminal secretion signal peptide. It is actively secreted and can be detected in culture media (Figure 14a). After PM20D1 overexpression, a significant decrease of soluble Aβ has been observed, with respect to control-infected neuronal culture (Figure 14b), indicating that PM20D1 acts as a direct or indirect Aβ-degrading enzyme.

Following the above described surprising observations, diagnostic methods to determine a state of AD are here described. A novel therapeutic approach is also described. These methods comprise the steps of placing a sample onto a substrate labeling the sample to identify at least one epigenetic marker and/or genetic variation; performing a multivariate statistical analysis to produce an output value; comparing the output value to a reference value; and determining a state of Alzheimer's disease, wherein one of said epigenetic markers is PM20D1 promoter DNA methylation and/or one of said genetic variation is at least one PM20D1 mQTL-associated SNPs , wherein said SNPs are selected from rs708727 and/or rs947211 and/or rs708730 and/or rs708724 and/or rs823130.

In a more preferred embodiment, said epigenetic marker is PM20D1 promoter DNA methylation, wherein an increase in methylation levels is associated with an AD diagnosis.

In a further preferred embodiment, said SNP is at rs708727, wherein the AA haplotype is associated with an AD diagnosis and/or at rs947211 wherein the GG haplotype is associated with an AD diagnosis and/or at rs708730 wherein the AA haplotype is associated with an AD diagnosis and/or at rs708724 wherein the CC haplotype is associated with an AD diagnosis and/or at rs823130 wherein the TT haplotype is associated with an AD diagnosis.

In a further embodiment, it is here described a method comprising the steps of identifying PM20D1 RNA expression in a sample; determining the expression level and comparing the value to a reference value; and determining a state of Alzheimer's disease. Optionally, said sample is genotype-corrected and, wherein said sample is a G rs708727 carrier, an increased PM20D1 RNA expression is indicative of AD.

In a further preferred embodiment, the genetic variation is a SNP at rs708727.

Still more preferably, the genetic variation is accompanied by an epigenetic marker that is an increased PM20D1 promoter DNA methylation.

Preferably, for the aim of the present invention, the sample is selected from one of cerebrospinal fluid, blood, serum, sputum, saliva, mucosal scraping, tissue biopsy, lacrimal secretion, semen, or sweat.

The presence of the one or more genetic variation is carried out by a process known to the man skilled in the art, preferably selected from the group consisting of direct sequencing, allele-specific probe hybridization, allele-specific primer extension, allele-specific amplification, allele-specific nucleotide incorporation, 5' nuclease digestion, molecular beacon assay, oligonucleotide ligation assay, size analysis, and single-stranded conformation polymorphism. In a further embodiment, electrophoresis, chromatography, mass spectroscopy, proteolytic digestion, protein sequencing, immunoaffinity assay, or a combination thereof are employed. Preferably, nucleic acids from the sample are amplified prior to determining the presence of the one or more genetic variation.

In a further preferred embodiment, the diagnostic method further comprises subjecting the subject to one or more additional diagnostic tests for AD selected from the group consisting of screening for one or more additional genetic/epigenetic markers, administering a mental status exam, or subjecting the subject to imaging procedures.

In a further embodiment, it is described a method of aiding prognosis of a sub phenotype of AD in a subject, the method comprising detecting in a biological sample derived from the subject the presence of a SNP at rs708727, wherein the G rs708727 carrier are characterized by increased expression levels of PM20D1 in a biological sample derived from the subject as compared to one or more control subjects.

In an embodiment, a viral vector codifying for PM20D1 is administered to a subject in need thereof. Alternatively, a recombinant PM20D1 protein is used.

The described approach is particularly suitable on a sub phenotype of AD characterized by a SNP at rs708727, wherein said sub phenotype of AD are subjects G carriers at rs708727.

In a further embodiment, the present application describes and claims the use of a kit comprising a set of molecular probes for diagnosis or prognosing AD comprising at least a probe capable of detecting directly or indirectly at least a marker selected from the group comprising: a SNP at rs708727 and/or at rs947211 and/or at rs708730 and/or rs708724 and/or rs823130. Preferably, said set further comprise one or more probes capable of detecting directly or indirectly a DNA methylation on PM20D1 promoter.

### Material and Method

### GWAS data

GWAS public data were obtained from NCBI GEO database (phs000168.v1.p1 NIA Late Onset AD and National Cell Repository for AD Family Study). Selected SNPs were extracted using PLIN v1.07 software (Purcell S et al., PLINK: a tool set for whole-genome association and population-based linkage analyses. Am J Hum Genet. 2007; 81(3):559-75) and the relation with AD by fitting bayesian univariate logistic regression using bayesglm (Gelman A, Su YS. 2013. arm: Data analysis using regression and multilevel/hierarchical models. R package version 1.6-09) with phenotype as outcome and asset point and interval estimates coding genotype as categorical variable and the allele-dosage test (p for trend) coding genotype as an ordered factor. Hardy-Weinberg equilibrium and allelic test was performed as previously reported (Guedj M et al., A fast, unbiased and exact allelic test for case-control association studies. Human Heredity 2006, 61:210-221). Linkage-disequilibrium data were obtained from SNAP Broad Institute database (https://www.broadinstitute.org/mpg/snap/ldsearch.php) and UCSC tracks for HapMap Release 24 CEU recombination map and HapMap Linkage Disequilibrium -Phase II from Phased Genotypes. Layered H3K27ac profiles were obtained from UCSC H3K27ac7 cell lines ENCODE track.

### EWAS data

EWAS public data were obtained from NCBI GEO database (GSE45775 and GSE57361) and analyzed using R software (http://www.Rproject.org) as previously described (Sanchez-Mut JV et al., 2015 previously cited). Briefly, GSE45775 Infinium HumanMethylation27 BeadArray 27K (Illumina) data were quantile normalized using lumi package and analyzed with Genefilter package (Bioconductor). DMRs were defined by two or more probes consistently reporting changes of DNA methylation. GSE57361 whole-genome bisulfite sequencing data were aligned using Bismark software (Krueger F and Andrews SR, Bismark: a flexible aligner and methylation caller for Bisulfite-Seq applications. Bioinformatics. 2011; 27(11):1571-2) and analyzed with SAMtools (Li H et al., The Sequence Alignment/Map format and SAMtools. Bioinformatics. 2009; 25(16):2078-9), Bedtools (Quinlan AR and Hall IM, BEDTools: a flexible suite of utilities for comparing genomic features. Bioinformatics 2010, 26(6):841-842), Tabix (Li H Tabix: fast retrieval of sequence features from generic TAB-delimited files. Bioinformatics 2011, 27(5):718-719), and ggplot2 packages. DMRs were identified by seeking regions with more than five consecutive CpG sites consistently located outside the 95% confidence interval of the smoothed methylation profile.

### Brain samples

Post-mortem tissues were obtained from the IDIBELL Biobank. Samples were dissected and characterized for Braak stage before further examination. DNA and RNA from grey matter samples of frontal cortex were extracted for subsequent experiments. Only samples with RIN > 6.5 according to the RNA quality test developed using the Agilent 2100 bio analyzer were included in the study. From these selected samples DNA and RNA from grey matter of frontal cortex (Brodmann area 9) were obtained, from 22 controls (Braak 0---II; 32% female; age 64 ± 3 years, mean ± SEM) and 23 Alzheimer's disease (Braak V---VI; 43% female; age 77 ± 2 years, mean ± SEM), wherein samples were age and gender matched.

### DNA methylation

DNA was isolated from post-mortem human brain samples by phenol-chloroform extraction and bisulphite converted using the EZ DNA methylation kit (Zymo Research) according to Bibikova et al. (2009) with minor modifications. Primers for bisulfite cloning/sequencing were designed using the Methyl Primer Express, version 1.0 (Applied Biosystems), and PCR products cloned in the pGEMT-easy sequencing vector (Promega) and Sanger-sequenced. Primers for pyrosequencing were designed using PyroMark assay design program, version 2.0.01.15 (Qiagen), and pyrosequencing reactions analyzed using PyroMark Q24 System version 2.0.6 (Qiagen) following manufacturer instructions.

### RNA expression

Total RNA purification and DNase treatment were performed using TRIzol (Invitrogen) and the Turbo DNA Free kit (Ambion). RNA was reverse transcribed using the Thermoscript RT-PCR system (Invitrogen), and PCR run using StepOnePlus RealTime PCR System (Applied Biosystems) and SYBR Green PCR MasterMaster Mix (Applied Biosystems). PCR efficiencies were calculated using standard dilutions and the LinReg software (Ruijter et al., 2009) and three housekeeping genes (GUSB, RPL38 and TBP) for normalizing PCR signals.

### Chromosome Conformation Capture

Chromatin conformation capture protocol (3C) was adapted from Court et al., 2011. In brief, cells were crosslinked 5 min with formaldehyde 2%, blocked 5 min with Glycine 125 mM, and lysed using an hypotonic buffer solution (Sucrose 0.25 M, KCl 25 mM, MgCl2 5 mM, TrisHCl 20 mM, pH 7.5). Nuclei were resuspended in CutSmart Buffer (New England Biolabs) and permeabilized with Triton X100 0.2 %. Chromatin was digested overnight with HindIII (New England Biolabs) and ligated overnight in diluted condition with T4 DNA ligase (New England Biolabs). Crosslinks were then reversed overnight at 65° C with 200mM NaCl and protein K treated. DNA was phenol-chloroform purified, ethanol precipitated, and resuspended in TE. BAC RRP11-219P13 (CHORI) was used for assaying all potential 3C products and primer PCR efficiencies, and efficiency of proximity ligation and DNA concentration for normalizing 3C signals.

### Chromatin immunoprecipitation assay

ChIP experiments were adapted from Gräff et al., 2012. Briefly, cells were crosslinked 10 min with formaldehyde 1 %, blocked 5 min with Glycine 125 mM, and cytoplasm lysed using cell lysis buffer (HEPES 5 mM, KCl 85 mM, NP40 0.5 %, pH 8). Nuclei were lysed with nuclear lysis buffer (TrisHCl 50 mM, EDTA 10 mM, SDS 1%, pH 8) and sonicated to -300 bp average size with EpiSonic Multi Functional Bioprocessor 1100 (EpiGentek). A total of 5-20 µg per sample and ChIP reaction was immunoprecipitated overnight at 4° C with H3K9ac (abcam), H3K14ac (abcam), H3K27ac (abcam), H4K12ac (abcam), CTCF (Millipore), MeCP2 (Sigma), and PolymeraseII (abcam) antibodies in diluted condition. Immunoprecipitated chromatin was then isolated using IgG Dynabeads (Life Sciences), and washed twice with low salt buffer (NaCl 150 mM, SDS 0.1 %, NP40 1 %, EDTA 1 mM, DeoNa 0.5 %, TrisHCl 50 mM, pH 8), high salt buffer (NaCl 500 mM, SDS 0.1 %, NP40 1 %, EDTA 1 mM, DeoNa 0.5 %, TrisHCl 50 mM, pH 8), lithium buffer (LiCl 2250 mM, SDS 0.1 %, NP40 1 %, EDTA 1 mM, DeoNa 0.5%, TrisHCl 50 mM, pH 8), and TE buffer (TrisHCl 10 mM, EDTA 0.25 mM, pH 8). Chromatin complexes were eluted with a solution of NaHCO₃ 0.1 M and SDS 1 %, and decrosslinked overnight at 65° C with 200 mM NaCl and proteinase K. DNA was phenol-chloroform extracted, ethanol precipitated, and resuspended in 50 µl TE. PCR was carried out in triplicate using SYBR Green PCR MasterMaster Mix (Applied Biosystems). Thermocycling conditions: 10 min 95°C; 50 cycles, 15 s 95°C; 1 min 60°C. Fluorescent signals were acquired by the StepOnePlus Real Time PCR System (Applied Biosystems). Values were normalized to total DNA input.

### Cloning

Human PM20D1 (AK057131.1) was cloned from a library of oligodT cDNA derived from SH SY5Y neuroblastoma cells. Rs708727 enhancer was cloned from immortalized B cells DNA. For PM20D1, forward 5'---AAAAGAATTCGCCACCATGGCTCAGCGGTGC---3' (SEQ ID No. 1) and Reverse 5'-CAAGGGATCCAGATGTCGGGAGGAAGGG---3' (SEQ ID No. 2) primers, containing EcoRI and NotI digestion sites respectively, were used. For rs708727 enhancer, were used the forward 5'-AGTAGGTACCCTTAGCTTAGCCATGTCATAGCCTTC---3' (SEQ ID No. 3) and reverse 5'-CGACGCGTCGATATTGGTTTACTCTCGTATCCTTAAAAAG---3' (SEQ ID No. 4) primers, containing KpnI and MluI digestion sites, respectively. Transgenes were amplified with high fidelity Prime STAR Max DNA Polymerase (Takara) according to manufacturer's instructions. PCR products were cloned in pGEMT easy (Promega) for amplifying the yield. Inserts were released by y EcoRI and NotI, and KpnI and MluI (New England Biolabs) digestions and ligated into pLVX-IRES ZsGreen1vector (Promega) and pGL3-promoter Luciferase Reporter Vector (Promega), respectively. All cloned plasmids were verified by Sanger sequencing.

### Luciferase assays

HEK293T cells were transfected with pGL3-promoter Luciferase reporter vector (Promega) containing mock or rs708727-enhancer using Lipofectamine 2000 (Thermo Fisher Scientific). After two days, cells were lysed and enhancer activity measured using Luciferase Assay System (Promega E1500) according to manufacturer instructions.

### Lentiviral production

Lentiviral vectors were produced in HEK293T cells using a third generation packaging system by calcium phosphate transfection with the following plasmids: pMD2.G (2.5 µg), psPAX2(7.5 ug) and pLVX-IRES ZsGreen1vector containing PM20D1/mock sequence (10 µg) . After four days, medium was collected and centrifuged in a SW32Ti ultracentrifuge rotor at 19,000 rpm for 90 min at 4°C. The pellet was resuspended in 120 µl of buffer containing 1× PBS pH 7.4 and 0.5% BSA for a 50X concentrated virus stock. Viral titer was determined using the HIV---1 p24 antigen ELISA kit (Zeptometrix Corp).

## Claims

1. A method for the diagnosis or prognosis of Alzheimer's disease, comprising the steps of placing a sample isolated from a person suspected of having Alzheimer's disease onto a substrate labeling the sample to identify at least one epigenetic marker and/or genetic variation and determining a state of Alzheimer's disease, wherein one of said epigenetic markers is an increased or decreased PM20D1 promoter DNA methylation and/or one of said genetic variation is in at least one of the PM20D1 mQTL-associated SNPs, which is a SNP at rs708727 and/or rs947211 and/or rs708730 and/or rs708724 and/or rs823130.

2. The method according to claim 1, wherein said at least one genetic variation is a AA allele at rs708727 and/or a GG allele at rs947211 and/or a AA allele at rs708730 and/or a CC allele at rs708724 and/or a TT allele at rs823130.

3. The method according to claim 1 or 2, wherein said at least one genetic variation is a SNP at rs708727.

4. The method according to any one of the claims from 1 to 3, wherein the sample is selected from one of cerebrospinal fluid, blood, serum, sputum, saliva, mucosal scraping, tissue biopsy, lacrimal secretion, semen, or sweat.

5. The method according to any one of the claims from 1 to 4, wherein the presence of the one or more genetic variation is carried out by a process selected from the group consisting of direct sequencing, allele-specific probe hybridization, allele-specific primer extension, allele-specific amplification, allele-specific nucleotide incorporation, 5' nuclease digestion, molecular beacon assay, oligonucleotide ligation assay, size analysis, single-stranded conformation polymorphism, electrophoresis, chromatography, mass spectroscopy, proteolytic digestion, protein sequencing, immunoaffinity assay, or a combination thereof.

6. The method according to any one of the claims from 1 to 5, further comprising subjecting the subject to one or more additional diagnostic tests for Alzheimer's disease selected from the group consisting of screening for one or more additional genetic/epigenetic markers, administering a mental status exam, or subjecting the subject to imaging procedures.

7. A viral vector encoding PM20D1, and/or a recombinant PM20D1 protein, for use in the treatment of Alzheimer's disease.

8. A viral vector and/or a recombinant PM20D1 protein for use according to claim 7, wherein said treatment is administered on a subphenotype of Alzheimer's disease, wherein said subphenotype of Alzheimer's disease are subjects G carriers at rs708727.

9. Use of a kit for diagnosing or prognosing Alzheimer's disease according to the method of claim 1, the kit comprising at least a probe capable of detecting directly or indirectly at least a genetic variation selected from the group comprising: a SNP at rs708727 and/or at rs947211 and/or at rs708730 and/or at rs708724 and/or rs823130.

10. The use according to claim 9, the kit further comprising one or more probes capable of detecting directly or indirectly a DNA methylation on PM20D1 promoter.

## Patentansprüche

1. Verfahren zur Diagnose oder Prognose der Alzheimer Krankheit, umfassend die Schritte eines Platzierens einer von einer Person, bei der der Verdacht auf Alzheimer Krankheit besteht, isolierten Probe auf einem Substrat, das die Probe markiert, um mindestens einen epigenetischen Marker und/oder eine genetische Variation zu identifizieren, und eines Bestimmens eines Zustandes der Alzheimer Krankheit, wobei einer der epigenetischen Marker eine erhöhte oder verringerte PM20D1-Promotor-DNA-Methylierung ist und/oder eine der genetischen Variationen in mindestens einem der PM20D1-mQTL-assoziierten SNPs vorliegt, die ein SNP an rs708727 und/oder rs947211 und/oder rs708730 und/oder rs708724 und/oder rs823130 ist.

2. Verfahren nach Anspruch 1, wobei die mindestens eine genetische Variation ein AA-Allel an rs708727 und/oder ein GG-Allel an rs947211 und/oder ein AA-Allel an rs708730 und/oder ein CC-Allel an rs708724 und/oder ein TT-Allel an rs823130 ist.

3. Verfahren nach Anspruch 1 oder 2, wobei die mindestens eine genetische Variation ein SNP an rs708727 ist.

4. Verfahren nach einem der Ansprüche 1 bis 3, wobei die Probe ausgewählt ist von einem von Zerebrospinalflüssigkeit, Blut, Serum, Sputum, Speichel, Schleimhautabstrich, Gewebebiopsie, Tränensekret, Sperma oder Schweiß.

5. Verfahren nach einem der Ansprüche 1 bis 4, wobei das Vorhandensein der einen oder mehreren genetischen Variationen durch einen Prozess durchgeführt wird, der aus der Gruppe ausgewählt ist, bestehend aus direkter Sequenzierung, allelspezifischer Sondenhybridisierung, allelspezifischer Primerverlängerung, allelspezifischer Amplifikation, allelspezifischem Nukleotideinbau, 5'-Nuklease-Verdau, molekularem Beacon-Assay, Oligonukleotid-Ligations-Assay, Größenanalyse, einzelsträngigem Konformationspolymorphismus, Elektrophorese, Chromatographie, Massenspektroskopie, proteolytischem Verdau, Proteinsequenzierung, Immunaffinitätsassay, oder einer Kombination davon.

6. Verfahren nach einem der Ansprüche 1 bis 5, ferne umfassend ein Unterziehen des Subjekts einem oder mehreren zusätzlichen diagnostischen Tests für die Alzheimer Krankheit, ausgewählt aus der Gruppe bestehend aus Screening auf einen oder mehrere zusätzliche genetische/epigenetische Marker, Vollziehen einer mentalen Statusuntersuchung oder Unterziehen des Subjekts bildgebender Verfahren.

7. Viraler Vektor, der für PM20D1 und/oder ein rekombinantes PM20D1-Protein kodiert, zur Verwendung bei der Behandlung der Alzheimer Krankheit.

8. Viraler Vektor und/oder rekombinantes PM20D1-Protein zur Verwendung nach Anspruch 7, wobei die Behandlung auf einen Subphänotyp der Alzheimer Krankheit angewendet wird, wobei der Subphänotyp der Alzheimer Krankheit Subjekte G Träger an rs708727 sind.

9. Verwendung eines Kits zur Diagnose oder Prognose der Alzheimer Krankheit nach dem Verfahren nach Anspruch 1, wobei das Kit mindestens eine Sonde umfasst, die in der Lage ist, direkt oder indirekt mindestens eine genetische Variation zu detektieren, ausgewählt aus der Gruppe umfassend: einem SNP an rs708727 und/oder an rs947211 und/oder an rs708730 und/oder an rs708724 und/oder rs823130.

10. Verwendung nach Anspruch 9, wobei das Kit ferner eine oder mehrere Sonden umfasst, die in der Lage sind, direkt oder indirekt eine DNA-Methylierung am PM20D1-Promotor nachzuweisen.

## Revendications

1. Procédé de diagnostic ou de pronostic de la maladie d'Alzheimer, comprenant les étapes consistant à placer un échantillon isolé provenant d'une personne suspectée d'être atteinte de la maladie d'Alzheimer sur un substrat marquant l'échantillon afin d'identifier au moins un marqueur épigénétique et/ou une variation génétique, et à déterminer un état de maladie d'Alzheimer, dans lequel l'un desdits marqueurs épigénétiques est une augmentation ou une diminution de la méthylation de l'ADN du promoteur de PM20D1 et/ou l'une desdites variations génétiques se trouve dans au moins un des SNP associés à un mQTL de PM20D1, qui est un SNP sur rs708727 et/ou rs947211 et/ou rs708730 et/ou rs708724 et/ou rs823130.

2. Procédé selon la revendication 1, dans lequel ladite variation génétique, au moins au nombre de une, est un allèle AA sur rs708727 et/ou un allèle GG sur rs947211 et/ou un allèle AA sur rs708730 et/ou un allèle CC sur rs708724 et/ou un allèle TT sur rs823130.

3. Procédé selon la revendication 1 ou 2, dans lequel ladite variation génétique, au moins au nombre de une, est un SNP sur rs708727.

4. Procédé selon l'une quelconque des revendications 1 à 3, dans lequel l'échantillon est sélectionné parmi un des éléments liquide céphalo-rachidien, sang, sérum, expectorations, salive, frottis de muqueuse, biopsie tissulaire, sécrétion lacrymale, sperme et sueur.

5. Procédé selon l'une quelconque des revendications 1 à 4, dans lequel la présence de la ou des variations génétiques est effectuée par un processus sélectionné dans le groupe constitué d'un séquençage direct, d'une hybridation de sonde spécifique d'un allèle, d'une extension d'amorce spécifique d'un allèle, d'une amplification spécifique d'un allèle, d'une incorporation de nucléotide spécifique d'un allèle, d'une digestion par une 5'-nucléase, d'un dosage de balises moléculaires, d'un dosage de ligature d'oligonucléotides, d'une analyse de taille, d'un polymorphisme de conformation simple brin, d'une électrophorèse, d'une chromatographie, d'une spectroscopie de masse, d'une digestion protéolytique, d'un séquençage de protéine, d'un dosage par immunoaffinité, ou d'une combinaison de ceux-ci.

6. Procédé selon l'une quelconque des revendications 1 à 5, comprenant en outre l'étape consistant à soumettre le sujet à un ou plusieurs tests de diagnostic supplémentaires de la maladie d'Alzheimer sélectionnés dans le groupe constitué du dépistage d'un ou de plusieurs marqueurs génétiques/épigénétiques supplémentaires, de la réalisation d'un examen d'état mental, ou consistant à soumettre le sujet à des procédures d'imagerie.

7. Vecteur viral codant pour PM20D1, et/ou une protéine PM20D1 recombinante, pour une utilisation dans le traitement de la maladie d'Alzheimer.

8. Vecteur viral et/ou protéine PM20D1 recombinante pour une utilisation selon la revendication 7, dans lequel ledit traitement est administré à un sous-phénotype de maladie d'Alzheimer, dans lequel ledit sous-phénotype de maladie d'Alzheimer est des sujets porteurs G sur rs708727.

9. Utilisation d'un kit pour diagnostiquer ou pronostiquer la maladie d'Alzheimer selon le procédé de la revendication 1, le kit comprenant au moins une sonde capable de détecter directement ou indirectement au moins une variation génétique sélectionnée dans le groupe comprenant : un SNP sur rs708727 et/ou rs947211 et/ou rs708730 et/ou rs708724 et/ou rs823130.

10. Utilisation selon la revendication 9, le kit comprenant en outre une ou plusieurs sondes capables de détecter directement ou indirectement une méthylation de l'ADN sur le promoteur de PM20D1.
